# EUROPÄISCHE PATENTANMELDUNG

(11) **EP 1 020 757 A1**
(43) Veröffentlichungstag der Anmeldung: **19.07.2000**
(21) Anmeldenummer: 99100342.7
(22) Anmeldetag: 14.01.1999
(51) Int. Cl.: G03B 42/04, A61B 6/14

(54) **Intraorale Halterung für einen Sensor**

(71) Anmelder: Schäfer, Klaus, Dr. med. dent., 59067 Hamm (DE)
(72) Erfinder: Schäfer, Klaus, Dr. med. dent., 59067 Hamm (DE)
(74) Vertreter: Thul, Hermann, Dipl.-Phys.

(57) **Zusammenfassung**

Die Erfindung betrifft eine intraorale Halterung (10) mit einem Bißkörper, wobei die Halterung (10) zu einer Lagerung eines Sensors (30) dient.

Erfindungsgemäß zeichnet sich die Halterung (10) dadurch aus, daß sie den Sensor (30) in einer verschiebbaren Position aufnehmen kann.

## Beschreibung

Die Erfindung betrifft eine intraorale Halterung mit einem Bißkörper, wobei die Halterung zu einer Lagerung eines Sensors dient.

Durch offenkundige Vorbenutzung sind Haltesysteme für Röntgensysteme bekannt, durch die sich mit Röntgenfilmen verschiedene Röntgenaufnahmetechniken realisieren lassen. Bei den Röntgenaufnahmetechniken handelt es sich insbesondere um die sogenannte Rechtwinkel-/Paralleltechnik, eine Kombination aus einer Rechtwinkeltechnik und einer Paralleltechnik. Die Rechtwinkel-/Paralleltechnik ermöglicht Röntgenaufnahmen mit einem Zahnfilm innerhalb der Mundhöhle.

Bei der Paralleltechnik werden Zahnachse und Filmachse parallel zueinander ausgerichtet. Trifft ein Zentralstrahl einer Röntgenquelle, insbesondere einer Röntgenröhre, auf die Mitte des Zahnes, trifft er ebenso auf die Mitte des Filmes. Die parallele Ausrichtung von Zahnachse und Filmachse gewährleistet eine Ablichtung des Zahnes auf den Film in einer im wesentlichen unveränderten Größe. Diese Aufnahmetechnik ist jedoch mit dem Nachteil einer ungenauen Justierung des Röntgentubus auf die Filmmitte sowie einer ungenauen Winkelstellung des Tubus zu dem Zahn und dem Film behaftet.

Bei der Rechtwinkeltechnik wird die Halterung so gestaltet, daß sie mit einem Tubus starr verbunden ist, so daß der Film im rechten Winkel zu dem Zentralstrahl des Röntgengerätes fixiert ist. Hierdurch trifft der Zentralstrahl stets im Winkel von 90° die Filmmitte. Diese Röntgenaufnahmetechnik hat den Vorteil, daß eine sichere Einhaltung eines rechten Winkels zwischen Zentralstrahl und Filmebene realisiert werden kann.

Bei der Rechtwinkel-/Paralleltechnik erfolgt zunächst eine Justierung des Filmes parallel zur Zahnachse und anschließend eine Ausrichtung des Tubus entlang einer Führungsstange. Hierdurch tritt der Zentralstrahl nahezu exakt im Winkel von 90° auf den Zahnfilm auf.

Der Erfindung liegt die Aufgabe zugrunde, eine Halterung für einen Sensor zu schaffen, wobei die Halterung eine Ablichtung eines oder mehrerer Zähne auf den Sensor in einer im wesentlichen unveränderten Größe ermöglicht und wobei eine möglichst genaue Justierung des Röntgentubus zu dem Sensor sowie eine genaue Winkelstellung des Röntgentubus zu dem Sensor erzielt wird. Insbesondere soll die Halterung sich für Aufnahmen von Wurzelkanälen eines Zahnes und gegebenenfalls mehrerer Zähne eignen.

Erfindungsgemäß wird diese Aufgabe dadurch gelöst, daß eine gattungsgemäße Halterung so gestaltet wird, daß sie den Sensor in einer verschiebbaren Position aufnehmen kann.

Die Halterung ist sowohl bei Aufnahmetechniken einsetzbar, bei denen eine genaue Größe des oder der Zähne unmittelbar durch flächengetreue Projektion erzielt wird, als auch bei Aufnahmetechniken, bei denen eine gleichbleibende Größe erst durch eine nachfolgende Bildverarbeitung erzielt wird.

Mit Hilfe der erfindungsgemäßen Halterung ist es möglich, Röntgenstrahlen außerhalb des Sensors wirksam abzuschirmen.

Insbesondere zum Zweck des erleichterten Einbringens der Halterung in die Mundhöhle kann der Sensor in der Halterung verschiebbar gelagert sein.

Die verschiebbare Lagerung des Sensors läßt sich besonders zweckmäßig dadurch erzielen, daß die Halterung wenigstens eine federnde Zunge aufweist. Hierdurch wird auf eine einfache und zweckmäßige Weise ein Formschluß erzielt.

Vorteilhafterweise ist die Halterung so gestaltet, daß das Haltemittel einen Rahmen mit einer Innenfläche zur Aufnahme des Sensors aufweist.

Eine besonders zweckmäßige Ausführungsform der Halterung zeichnet sich dadurch aus, daß die Innenfläche des Rahmens sich im wesentlichen parallel zu dem Bißkörper erstreckt. Röntgenaufnahmen eines Behandlungszustands des Zahnes, insbesondere seiner Zahnwurzelkanäle, lassen sich bevorzugt dadurch erreichen, daß die Halterung so gestaltet ist, daß der Bißkörper ein Hohlkörper ist, einen Hohlkörper aufweist oder mit einem Hohlkörper verbunden ist. Bei anderen Einsatzgebieten kann der Bißkörper jedoch auch eine andere Gestalt aufweisen.

Es ist zweckmäßig, daß der Hohlkörper eine Höhe von etwa 1 cm bis 2 cm, vorzugsweise etwa 1,5 cm aufweist.

Ein in einen Wurzelkanal eines Zahnes eingeführtes Behandlungsgerät, insbesondere ein Wurzelkanal-Aufbereitungsinstrument (Reamer), wird aufgrund des Hohlraumes des Bißkörpers (Aufbißblocks) so geschützt, daß eine Röntgenaufnahme des Zahnes problemlos erfolgen kann, ohne daß ein Griff des Behandlungsgerätes durch Zubeißen des Patienten weiter als gewünscht in den Zahn hineingedrückt wird. Somit endet der Griff auch während des Röntgens in diagnostisch exakter Position oberhalb der Zahnkrone.

Zur Aufnahme des Behandlungsgerätes ist der Aufßbißblock als ein Hohlkörper geformt.

Ein wirksamer Schutz von Patienten vor Röntgenstrahlung läßt sich dadurch erzielen, daß die Halterung mit einem Befestigungsmittel für eine mögliche Befestigung einer Abschirmblende und/oder für eine mögliche Befestigung eines Visierringes verbindbar ist. Durch den Visierring kann eine im Inneren des Röntgentubus befindliche Blende zur Eingrenzung des Nutzstrahlbündels entsprechend der Sensorfläche eingestellt werden.

Vorzugsweise sind das Befestigungsmittel und die Abschirmblende so gestaltet, daß eine Öffnung der Abschirmblende im wesentlichen parallel zu dem Sensor ausrichtbar ist.

Weitere Vorteile, Besonderheiten und zweckmäßige Weiterbildungen der Erfindung ergeben sich aus der nachfolgenden Darstellung eines bevorzugten Ausführungsbeispiels anhand der Zeichnungen.

Von den Zeichnungen zeigt:

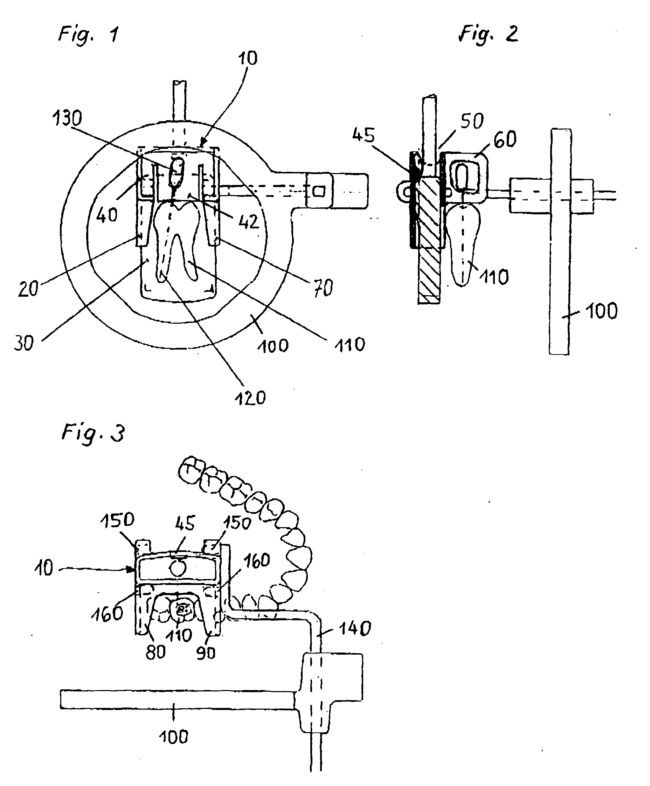

- Fig. 1: eine Vorderansicht einer Halterung 10 in einer sich parallel zu einem Sensor 30 erstreckenden Ebene,
- Fig. 2: einen Querschnitt durch die in Fig. 1 dargestellte Halterung 10 und
- Fig. 3: eine Draufsicht auf die in den Fig. 1 und 2 dargestellte Halterung 10.

Eine Halterung 10 weist einen Rahmen 40 auf, der einen Sensor 30 aufnehmen kann. Zur Aufnahme des Sensors 30 dient eine Öffnung 50 des Rahmens 40. Zweckmäßigerweise erstreckt sich eine Ebene der Öffnung des Rahmens 40 im wesentlichen parallel zu einem Bißkörper 60.

Der Rahmen 40 weist eine elastische Zunge 42 auf, die zu einem Festklemmen des Sensors 30 dient.

Der Rahmen 40 weist verlängerte Führungen 20 und 70 auf, die den Sensor 30 zusätzlich zu einer Haltefunktion des Rahmens 40 durch Formschluß halten. Die Führungen 20 und 70 sind vorzugsweise mit seitlichen Flanken versehen, so daß der Formschluß verstärkt wird.

Die Öffnung 50 kann eine überwindbare Arretierung 45 aufweisen, so daß der Sensor 30 in arretierter Position gegenüber dem Visierring 100 justiert werden kann. Hierdurch ist es insbesondere möglich, daß der Mittelpunkt des Sensors 30 in der arretierten Position mit dem Mittelpunkt des Visierrings 100 übereinstimmt.

Der Bißkörper 60 weist Ausnehmungen auf, so daß er einen Hohlkörper bildet. Vorzugsweise weist der Bißkörper 60 eine im wesentlichen rechteckige Form auf. Der Hohlkörper enthält Strukturelemente wie Vorsprünge 80, 90, um zu verhindern, daß ein Patient während einer Röntgenaufnahme auf innerhalb des Hohlkörpers befindliche Bearbeitungsinstrumente beißt. Die Höhe des Bißkörpers 60 (Aufbißblock) beträgt vorzugsweise ungefähr 1 cm bis 2 cm, vorzugsweise etwa 1,5 cm.

In einen Zahnwurzelkanal 120 des Zahns 110 kann ein geeignetes Behandlungsmittel 130 eingeführt werden.

Die Halterung 10 ist mit einem Visierring 100 verbindbar. Der Visierring 100 ermöglicht eine genaue Justierung eines Röntgentubus auf die Mitte des Sensors 30 und gleichzeitig auf einen zu behandelnden Zahn 110.

Eine Verbindung der Halterung 10 mit dem Visierring 100 erfolgt vorzugsweise über wenigstens ein Führungsmittel 140. Die Halterung 10 und das Führungsmittel 140 sind lösbar miteinander verbunden. Eine besonders einfache Verbindung zwischen der Halterung 10 und dem Visierring 100 läßt sich dadurch erzielen, daß die Halterung 10 wenigstens zwei Ausnehmungen 150, 160 aufweist. In die Ausnehmungen 150, 160 können komplementär geformte Vorsprünge des Führungselementes 140 eingebracht werden. Bei dem Führungsmittel 140 handelt es sich im einfachsten Fall um eine Führungsstange.

### Bezugszeichenliste

- 10: Halterung
- 20: Führung
- 30: Sensor
- 40: Rahmen
- 42: elastische Zunge
- 45: elastische Arretierung
- 50: Öffnung
- 60: Bißkörper
- 70: Führung
- 80: Vorsprung
- 90: Vorsprung
- 100: Visierring
- 110: zu behandelnder Zahn
- 120: Zahnwurzelkanal
- 130: Behandlungsmittel
- 140: Führungsmittel
- 150: Ausnehmung
- 160: Ausnehmung

## Patentansprüche

1. Intraorale Halterung (10) mit einem Bißkörper (60), wobei die Halterung (10) für eine Lagerung eines Sensors (30) dient, **dadurch gekennzeichnet,** daß die Halterung (10) den Sensor (30) in verschiebbaren Positionen aufnehmen kann.

2. Halterung (10) nach Anspruch 1,
**dadurch gekennzeichnet,** daß die Halterung (10) wenigstens eine elastische Zunge (42) aufweist.

3. Halterung (10) nach einem der Ansprüche 1 oder 2, **dadurch gekennzeichnet,** daß die Halterung (10) einen Rahmen (40) mit einer Öffnung (50) zur Aufnahme des Sensors (30) aufweist.

4. Halterung (10) nach Anspruch 3, **dadurch gekennzeichnet,** daß eine Öffnungsebene des Rahmens (40) sich im wesentlichen parallel zu dem Bißkörper (60)erstreckt.

5. Halterung (10) nach einem der Ansprüche 1 bis 4, **dadurch gekennzeichnet,** daß der Bißkörper (60) Ausnehmungen aufweist.

6. Halterung (10) nach Anspruch 5,
**dadurch gekennzeichnet,** daß der Bißkörper (60) als ein Hohlkörper gestaltet ist.

7. Halterung (10) nach einem der Ansprüche 1 bis 6, **dadurch gekennzeichnet,** daß eine Höhe des Bißkörpers (60) ungefähr 1 cm bis 2 cm beträgt.

8. Halterung (10) nach einem der Ansprüche 1 bis 7, **dadurch gekennzeichnet,** daß sie mit einem Befestigungsmittel für eine Befestigung einer Abschirmblende verbindbar ist.

9. Halterung (10) nach einem der Ansprüche 1 bis 8, **dadurch gekennzeichnet,** daß das Befestigungsmittel so gestaltet ist, daß eine Öffnung der Abschirmblende im wesentlichen parallel zu dem Sensor (30) ausgerichtet ist.
